# EUROPEAN PATENT SPECIFICATION

(11) **EP 1 274 562 B1**
(45) Date of publication and mention of the grant of the patent: **07.07.2004**
(21) Application number: 01925044.8
(22) Date of filing: 16.04.2001
(51) Int. Cl.: B29D 11/00

(54) **METHOD FOR MAKING AN ACCOMMODATING INTRAOCULAR LENS**
METHODE ZUR HERSTELLUNG EINER AKKOMODIERFÄHIGEN INTRAOKULARLINSE
PROCEDE DE FABRICATION D'UNE LENTILLE INTRAOCULAIRE D'ACCOMMODATION

(30) Priority: 21.04.2000 US 522746
(43) Date of publication of application: 15.01.2003
(73) Proprietor: Advanced Medical Optics, Inc., Irvine, CA 92612 (US)
(72) Inventor: WEINSCHENK, Joseph, I., III, Fort Worth, TX 76132 (US)
(74) Representative: HOFFMANN - EITLE
(86) International application number: PCT/US2001/012404
(87) International publication number: WO 2001/081075

(56) References cited:
- EP-A- 0 331 457
- EP-A- 0 552 528
- EP-A- 0 920 980

## Description

### Background of the Invention

### 1. Field of the Invention

The present invention relates to the field of intraocular lens and, in particular, intraocular lenses that move axially to alleviate the condition of presbyopia when implanted in the eye of a patient.

### 2. Description of the Art

Presbyopia is the medical term for the condition in which an individual loses the ability to focus on nearby objects such as the words in a book.

Research is currently underway to address this medical condition. In particular, research is focused on the replacement of the "malfunctioning" natural lens with an artificial one that could move axially or change shape so that the patient would have restored accommodating ability.

An intraocular lens, i.e. an IOL, that moves axially when subjected to the muscular forces within the eye, will accommodate to deliver light to the proper spot on the retina. In other words, the IOL would deliver "focusable" vision by axial movement.

In such an IOL it is desirable for the lens to "bend" at the haptic-optic junction in order to achieve the axial movement required. However, since the compression force in the eye is known to be very weak, the material strength at the haptic-optic junction must be low.

Therefore, rigid IOL materials, comprising a single material for both the optic and the haptic, like polymethylmethacrylate or PMMA, would not be suitable for providing an IOL that would accommodate as described. Also, typical "soft" acrylic materials, which have a tensile strength about 840 psi, may also be too strong to provide both the optic and the haptic portions of an accommodating IOL. Conversely, known "soft" IOL materials like silicones or acrylic hydrogels are likely too soft and, therefore, a lens made with these types of materials, as the haptics, would not function as desired. Instead, the haptics of these lenses might bend anywhere from the haptic-optic junction to the lens periphery.

Methods for manufacturing intraocular lenses are described in EP-A-920,980, EP-A-552,528 and EP-A-331,457.

Thus, it is an object of the present invention to provide an accommodating IOL which will bend at the optic-haptic junction to alleviate presbyopia or other ocular conditions wherein light is not delivered to the proper spot on the retina.

### Brief Summary of the Invention

The present invention provides a method of making an intraocular lens having an optic portion joined to a haptic portion by a flexible element which comprises the steps of:
(a) forming a core of a material suitable for use as said optic portion;
(b) reacting said core to provide a first composite of said optic portion and said flexible element bonded thereto;
(c) reacting said flexible element with a material suitable for use as said haptic portion to provide a second composite; and
(d) machining said second composite to form said intraocular lens.

In one embodiment of the invention the first composite is provided by polymerizing a first polymerizable material capable of forming said flexible element to form an outer layer about said core.

In a second embodiment, the first composite is provided by reacting the core, at the surface thereof, e.g. by hydrolyzing the surface core material, to form said flexible element.

In said first embodiment, the core may be placed in a mold, said first polymerizable material may be placed in said mold about said core and said first polymerizable material may be polymerized around said core.

In either embodiment, a second polymerizable material, capable of forming said haptic portion, is polymerized about said first composite to form an outer layer about said first composite.

The second composite is preferably provided by positioning said first composite in a mold, placing a second polymerizable material in said mold about said first composite and polymerizing said second polymerizable material.

### Brief Description of the Drawings

Figure 1 shows the IOL prepared by the method of the present invention.
Figure 2 shows the method of forming the core used in preparing the above IOL.
Figure 3a and 3b show the methods for forming the first composite used in preparing the IOL of the present invention.
Figure 4 shows the method for preparing the second composite used in preparing the IOL of the present invention.
Figure 5a shows a "three piece" IOL as may be fabricated with the method of the present invention.
Figure 5b shows a "one piece" IOL, having a haptic skirt, as may be fabricated with the method of the present invention.

### Detailed Description of the Invention

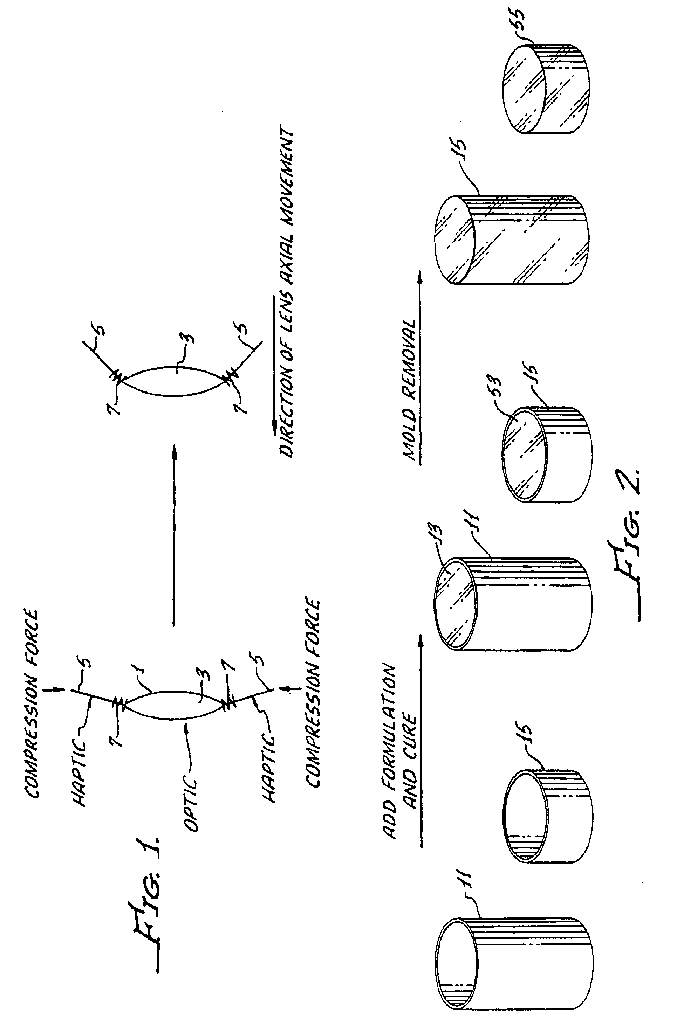

An intraocular lens 1 as shown in Figure 1 includes an optic 3 and one or more haptics 5 joined to said optic 3 by flexible portion 7 which is softer or more flexible than either the optic 3 or the haptics 5. As shown, when a compressive force is applied to the haptics 5, the optic 3 moves axially. Thus, when implanted in the eye of a patient, the optic may accommodate to focus light on the patient's retina.

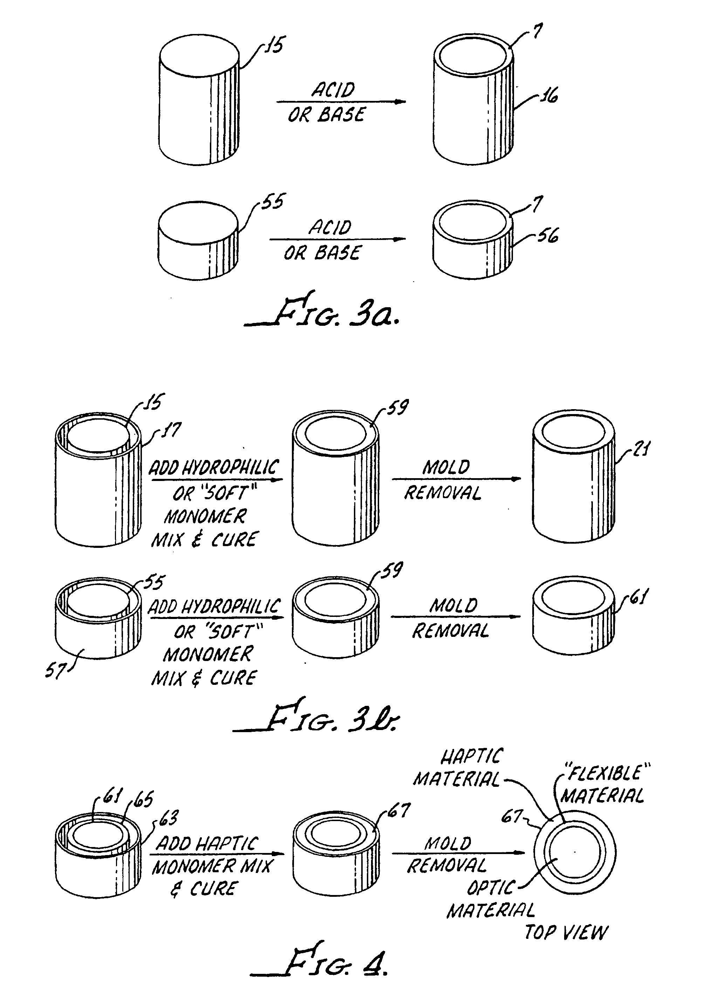

Intraocular lens 1 may be prepared as shown in Figures 2 through 4.

As shown in Figure 2, a tube 11 or cup 51 is utilized as a mold to prepare the core that is utilized as the center optic material in the finished IOL. An appropriate liquid monomer mixture, including monomers and an initiator, is placed in the respective tube or cup mold and cured with thermal or ultraviolet (UV) or another energy source, as known in the art, to yield a solid core as shown in 13 and 53.

A suitable monomer mixture for forming said core may include 99.5%, by weight, methylmethacrylate, and 0.5%, by weight, of a UV absorber such as benzotriazole. The molds are then removed to provide the core as a rod 15 or button 55.

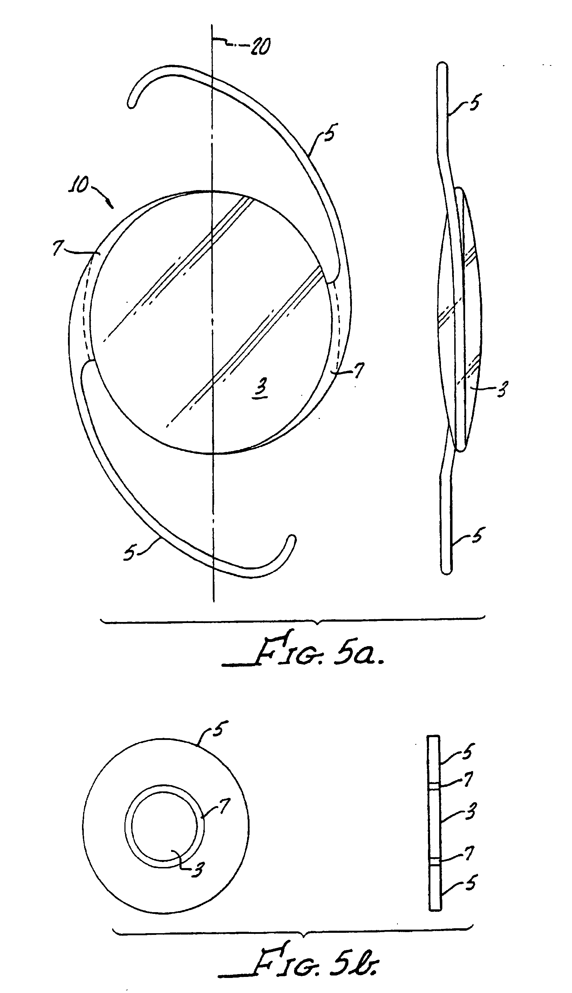

As shown in Figures 3a and 3b, the flexible element 7 that is affixed to the optic portion in the IOL of the invention, may be provided by one of two methods.

In the first method, as shown in Figure 3a, rod 15 or button 55 is treated with a strong acid or base to hydrolyze the surface thereof and thereby provide the first composite 16 or 56, respectively. In such hydrolysis, a portion of the surface ester groups on the methylmethacrylate homopolymer are converted to the corresponding carboxylic acid groups, as shown. The hydrolyzed surface will soften when contacted with aqueous liquids, thus, providing the flexible element in the IOL of the invention. A suitable strong base might be a 50%, by weight, aqueous NaOH solution. A suitable strong acid might be a 70%, by weight, aqueous H₂SO₄ solution. The hydrolysis may be continued until the surface of said rod 15 or button 55 is hydrolyzed to a depth of about 0.5 mm or more, depending on the desired thickness of the flexible element portion 7 of the first composite 16 or 56.

Alternatively, as shown in Figure 3b, rod 15 or button 55 is placed in the center of an oversized mold 17 and 57, respectively, and a hydrophilic or soft monomer mixture 19 and 59, respectively, is poured into the space between the mold and the rod 15 or button 55. The hydrophilic or soft monomer mixture is cured with the appropriate energy source as described above to convert rod 15 or button 55 into the first composite 21 or 61, respectively.

A suitable hydrophilic monomer mixture may include 99%, by weight, hydroxyethyl methacrylate (HEMA)and 1%, by weight, of a crosslinker, such as ethyleneglycol-dimethacrylate (EGDMA) as well as a peroxide or azo compound as an initiator. A suitable soft monomer mixture may include 99%, by weight, ethylacrylate (EA) and 1%, by weight, of a crosslinker such as EGDMA.

In this embodiment of the present invention, the flexible element 7, formed from the hydrophilic monomer mixture functions as the hydrolyzed surface of the first composite 16 or 56, discussed above. That is the polymer resulting from the hydrophilic monomer mixture softens when contacted with aqueous liquids to provide the flexible element 7. In contrast, polymer resulting from a soft monomer mixture is, itself, more flexible than the rod 15 or button 55 and the later added haptics to thereby bend as required in the accommodating IOL of the invention.

In said alternative method for preparing said first composite, it is preferable to allow the added hydrophilic or soft monomer mix to diffuse into the button 55 or rod 15 for a certain amount of time before initiating the cure. This results in an interpenetrating network between the core and hydrophilic or soft outer layer which becomes the flexible element, thus increasing the strength of the "bond" between them.

Finally, as shown in Figure 4, the first composite which is designated as 16, 56, 21 or 61 in the embodiments set forth in Figures 3a and 3b, is provided with an element for forming the haptic at the outer part of the first composite. In the final product, an IOL, this material has the desirable properties possessed by the haptics and/or "skirts" that surround the optic portion in one-piece IOL lens designs.

The first composite is placed in the center of an oversized mold 63 and then the liquid monomer mix 65 is added into the mold to fill the void around the first composite. The liquid monomer mix 65 is cured with the appropriate energy source, and then mold removal is effected to yield the second composite 67. This is illustrated for the first composite 61, only, in Figure 4, but the other examples of said first composite, i.e. 16, 56 or 21, could be treated in the same manner to form a haptic material thereabout.

An example rigid composition for the haptic material would be
100% MMA

An example flexible composition for the haptic material would be

| | |
|---|---|
| 58% | ethyl acrylate (EA) |
| 29% | ethyl methacrylate (EMA) |
| 9% | 2,2,2-trifluoroethyl methacrylate (TFEMA) |
| 3.5% | EGDMA |
| 0.5% | UV Absorber (e.g., benzotriazole) |

Manufacture of an IOL from said second composite 67 is effected by lathing the button-shaped first composite 67, shown in Figure 4, or by cutting a button from a first composite in the form of a rod, e.g. 16 or 21, first, and the resultant button being like that just noted in Figure 4, may, be lathed/milled to resemble a 3-piece IOL as shown in Figure 5a, or a one-piece IOL with a "skirt" as shown in Figure 5b.

While illustrated above for specific polymers that can be used to form the optic portion, the flexible element and the haptic portion, it will be appreciated that other polymers may be used provided only that the flexible element be softer than either the optic portion or the haptic portion so that the optic portion may move axially when subject to compressive force by the muscles of the implanted eye to focus light properly on the retina.

For example the optic portion may be one of the following polymers:
- poly(methyl methacrylate)
- copolymer of

| | |
|---|---|
| 2-phenoxyethyl acrylate | 70% |
| 2-phenoxyethyl methacrylate | 28% |
| EGDMA | 2% |

- copolymer of

| | |
|---|---|
| 2-phenoxyethyl acrylate | 32.5% |
| 2-phenoxyethyl methacrylate | 48.8% |
| 3,5,5-trimethylhexyl acrylate | 14.7% |
| Ethylene glycol dimethacrylate | 2.0% |
| UV absorber | 2.0% |

- copolymer of

| | |
|---|---|
| Ethyl acrylate | 55% |
| Ethyl methacrylate | 50% |
| 2,2,2-trifluoroethyl methacrylate | 10% |
| Ethylene glycol dimethacrylate | 3.5% |
| UV absorber | 1.5% |

- copolymer of crosslinked polydimethyldiphenylsiloxane
The flexible element may comprise one of the following polymers.
- copolymers, including a lightly crosslinked copolymers, comprised of acrylate and methacrylate monomers, and whose Tg is no greater than about 13° C, for example:
- copolymers, including a lightly crosslinked copolymers, of
Butyl acrylate or hexyl acrylate and
Methyl methacrylate or ethyl methacrylate
- copolymer, including a lightly crosslinked copolymer, of

| | |
|---|---|
| 2-hydroxyethyl methacrylate | 71% |
| N-vinyl pyrrolidone | 29% |

- copolymer, including a lightly crosslinked copolymer, of

| | |
|---|---|
| ethyl acrylate | 17% |
| 2-hydroxyethyl methacrylate | 69% |
| N-vinyl pyrrolidone | 14% |

- copolymer, including a lightly crosslinked copolymer, of

| | |
|---|---|
| ethyl acrylate | 6% |
| 2-phenoxyethyl acrylate | 29% |
| N-vinyl pyrrolidone | 59% |
| 3,5,5,-trimethylhexylmethacrylate | 6% |

- copolymer of

| | |
|---|---|
| ethyl acrylate | 59.7% |
| N-vinyl pyrrolidone | 39.8% |
| tetraethylene glycol dimethacrylate | 0.25% |

- copolymer of

| | |
|---|---|
| ethyl acrylate | 28.6% |
| methyl methacrylate | 14.3% |
| dimethyl acrylate | 56.8% |
| tetraethylene glycol dimethacrylate | 0.4% |

- copolymer of very lightly crosslinked polydimethyldiphenylsiloxane
The haptic may comprise one of the following polymers.
- poly(methylmethacrylate)
- crosslinked copolymer comprised of acrylate/methacrylate monomers with a Tg of at least 40° C.
- copolymer of crosslinked polydimethyldiphenylsiloxane

## Claims

1. A method of making an accommodating intraocular lens having an optic portion joined to a haptic portion by a flexible element which comprises the steps of:
(a) forming a core of a material suitable for use as said optic portion;
(b) reacting said core to provide a first composite of said optic portion and said flexible element bonded thereto;
(c) reacting said flexible element with a material suitable for use as said haptic portion to provide a second composite; and
(d) machining said second composite to form said intraocular lens.

2. The method of claim 1 comprising the step of polymerizing about said core a first polymerizable material capable of forming said flexible element to form an outer layer about said core.

3. The method of claim 2 further comprising positioning said core in a mold, placing said first polymerizable material in said mold about said core, and polymerizing said first polymerizable material.

4. The method of claim 3 further comprising the step of polymerizing about said first composite a second polymerizable material, capable of forming. said haptic portion, to form an outer layer about said first composite.

5. The method of claim 4 further comprising positioning said first composite in a mold, placing said second polymerizable material in said mold about said composite, and polymerizing said second polymerizable material.

6. The method of claim 1 wherein said optic portion comprises a high refractive material.

7. The method of claim 1 comprising the step of hydrolyzing the surface of said core to provide said flexible element.

8. The method of claim 7 further comprising the step of polymerizing about said first composite a polymerizable material, capable of forming said haptic portion, to form an outer layer about said first composite.

9. The method of claim 7 further comprising positioning said first composite in a mold, placing said polymerizable material in said mold about said composite, and polymerizing said polymerizable material.

10. The method of claim 7 wherein said surface is hydrolyzed by reaction with a strong acid or base.

11. The method of claim 6 wherein said high refractive material is selected from the group consisting of polymethylmethacrylate, and copolymers of high refractive index monomers selected from the group consisting of 2-phenoxyethyl acrylate, 2-phenoxyethyl methacrylate, benzyl acrylate, benzyl methacrylate, 2-phenylethyl acrylate, 2-phenylethyl methacrylate, N-vinyl carbazole, 3-phenylpropyl acrylate, and 3-phenylpropyl methacrylate.

12. The method of claim 2 wherein said flexible element is selected from the group consisting of poly(ethylacrylate) homopolymer, hydrated polyhydroxyethylacrylate, polymers and copolymers of n-propyl acrylate, n-butyl acrylate, n-hexyl acrylate, hydrated N-vinyl pyrrolidone, hydrated 3-hydroxypropyl methacrylate, hydrated methacrylic acid, hydrated acrylic acid, lightly crosslinked poly(dimethyldiphenylsiloxane), and lightly crosslinked poly(dimethylsiloxane).

13. The method of claim 1 wherein said haptic portion is selected from the group consisting of poly-(methylmethacrylate), crosslinked copolymers comprised of acrylate/methacrylate monomers with a Tg of at least 40° C and copolymers of crosslinked polydimethyldiphenylsiloxane.

14. The method of claim 1 wherein said flexible element is more flexible than said optic portion and said haptic portion.

15. The method of claim 1 wherein said optic portion is polymethylmethacrylate.

16. The method of claim 1 wherein said flexible element is selected from the group consisting of polyethylacrylate and hydrated polyhydroxyethylacrylate.

17. The method of claim 1 wherein said haptic portion is polymethylmethacrylate.

18. The method of claim 5 further comprising providing said second composite as a rod having said material suitable for use as said haptic portion as an outer layer around said first composite.

19. The method of claim 18 further comprising cutting a disc from said rod and removing a portion of said outer layer of said rod to form at least one haptic portion.

## Patentansprüche

1. Verfahren zur Herstellung einer akkomodierenden Intraokularlinse mit einem optischen Bereich, der mittels eines flexiblen Elements an einen haptischen Bereich gebunden ist, das die folgenden Schritte umfasst:
(a) Ausbildung eines Kerns aus einem Material, das zur Verwendung als optischer Bereich geeignet ist;
(b) Umsetzung des Kerns, wodurch ein erstes Komposit aus dem optischen Bereich und dem daran gebundenen flexiblen Element bereitgestellt wird;
(c) Umsetzen des flexiblen Elements mit einem Material, das zur Verwendung als haptischer Bereich geeignet ist, wodurch ein zweites Komposit bereitgestellt wird; und
(d) Bearbeiten des zweiten Komposits unter Ausbildung der Intraokularlinse.

2. Verfahren gemäss Anspruch 1, das den Schritt der Polymerisation eines ersten polymerisierbaren Materials, das zur Ausbildung des flexiblen Elements geeignet ist, um den Kern umfasst, wodurch eine äussere Schicht um den Kern gebildet wird.

3. Verfahren gemäss Anspruch 2, das ferner die Positionierung des Kerns in einer Form zum Plazieren des polymerisierbaren Materials in der Form um den Kern, und Polymerisation des ersten polymerisierbaren Materials umfasst.

4. Verfahren gemäss Anspruch 3, das ferner den Schritt des Polymerisierens eines zweiten polymerisierbaren Materials, das zur Ausbildung des haptischen Bereichs geeignet ist, um das erste Komposit umfasst, wodurch eine äussere Schicht um das erste Komposit gebildet wird.

5. Verfahren gemäss Anspruch 4, das ferner die Positionierung des ersten Komposits in einer Form, Plazieren des zweiten polymerisierbaren Materials in der Form um das Komposit, und Polymerisation des zweiten polymerisierbaren Materials umfasst.

6. Verfahren gemäss Anspruch 1, worin der optische Bereich ein hochbrechendes Material umfasst.

7. Verfahren gemäss Anspruch 1, das den Schritt der Hydrolyse der Oberfläche des Kerns unter Bereitstellung des flexiblen Elements umfasst.

8. Verfahren gemäss Anspruch 7, das ferner den Schritt der Polymerisation eines polymerisierbaren Materials, das zur Ausbildung des haptischen Bereichs geeignet ist, um das erste Komposit umfasst, wodurch eine äussere Schicht um das erste Komposit gebildet wird.

9. Verfahren gemäss Anspruch 7, das ferner die Positionierung des ersten Komposits in einer Form, Plazieren des zweiten polymerisierbaren Materials in der Form um das Komposit, und Polymerisation des zweiten polymerisierbaren Materials umfasst.

10. Verfahren gemäss Anspruch 7, worin die Oberfläche durch Umsetzen mit einer starken Säure oder Base hydrolysiert wird.

11. Verfahren gemäss Anspruch 6, worin das hochbrechende Material ausgewählt ist aus Polymethylmethacrylat und Copolymeren aus Monomeren mit hohem Brechungsindex, ausgewählt aus 2-Phenoxyethylacrylat, 2-Phenoxyethylmethacrylat, Benzylacrylat, Benzylmethacrylat, 2-Phenylethylacrylat, 2-Phenylethylmethacrylat, N-Vinylcarbazol, 3-Phenylpropylacrylat und 3-Phenylpropylmethacrylat.

12. Verfahren gemäss Anspruch 2, worin das flexible Element ausgewählt ist aus Poly(ethylacrylat)-Homopolymer, hydratisiertem Polyhydroxyethylacrylat, Polymeren und Copolymeren von n-Propylacrylat, n-Butylacrylat, n-Hexylacrylat, hydratisiertem N-Vinylpyrrolidon, hydratisiertem 3-Hydroxypropylmethacrylat, hydratisierter Methacrylsäure, hydratisierter Acrylsäure, leicht vernetztem Poly(dimethyldiphenylsiloxan) und leicht vernetztem Poly(dimethylsiloxan).

13. Verfahren gemäss Anspruch 1, worin der haptische Bereich ausgewählt ist aus Poly(methylmethacrylat), vernetzten Copolymeren aus Acrylat/Methacrylatmonomeren mit einem Tg-Wert von mindestens 40°C und Copolymeren aus vernetztem Polydimethyldiphenylsiloxan.

14. Verfahren gemäss Anspruch 1, worin das flexible Element flexibler ist als der optische Bereich und der haptische Bereich.

15. Verfahren gemäss Anspruch 1, worin der optische Bereich Polymethylmethacrylat ist.

16. Verfahren gemäss Anspruch 1, worin das flexible Element ausgewählt ist aus Polyethylacrylat und hydratisiertem Polyhydroxyethylacrylat.

17. Verfahren gemäss Anspruch 1, worin der haptische Bereich Polymethylmethacrylat ist.

18. Verfahren gemäss Anspruch 5, das ferner die Bereitstellung des zweiten Komposits als einen Stab, der das zur Verwendung als haptischer Bereich geeignete Material als eine äussere Schicht um das erste Komposit aufweist, umfasst.

19. Verfahren gemäss Anspruch 18, das ferner das Schneiden einer Scheibe aus dem Stab und Entfernen eines Bereichs der äusseren Schicht des Stabs unter Ausbildung von mindestens einem haptischen Bereich umfasst.

## Revendications

1. Procédé de fabrication d'une lentille intraoculaire d'accommodation ayant une partie optique reliée à une partie haptique par un élément flexible, comprenant les étapes suivantes :
(a) former une âme en un matériau pouvant être utilisé comme ladite partie optique ;
(b) mettre en réaction ladite âme pour fournir un premier composite de ladite partie optique et dudit élément flexible lié à celle-ci ;
(c) mettre en réaction ledit élément flexible avec un matériau pouvant être utilisé comme ladite partie haptique pour fournir un second composite ; et
(d) usiner ledit second composite pour former ladite lentille intraoculaire.

2. Procédé selon la revendication 1, comprenant l'étape de polymériser autour de ladite âme un premier matériau polymérisable capable de former ledit élément flexible pour former une couche externe autour de ladite âme.

3. Procédé selon la revendication 2, comprenant en outre le positionnement de ladite âme dans un moule, la mise en place dudit premier matériau polymérisable dans ledit moule autour de ladite âme, et la polymérisation dudit premier matériau polymérisable.

4. Procédé selon la revendication 3, comprenant en outre l'étape de polymériser autour dudit premier composite un second matériau polymérisable, capable de former ladite partie haptique, pour former une couche externe autour dudit premier composite.

5. Procédé selon la revendication 4, comprenant en outre le positionnement dudit premier composite dans un moule, la mise en place dudit second matériau polymérisable dans ledit moule autour dudit composite, et la polymérisation dudit second matériau polymérisable.

6. Procédé selon la revendication 1, dans lequel ladite partie optique comprend un matériau à réfraction élevée.

7. Procédé selon la revendication 1, comprenant l'étape d'hydrolyse de la surface de ladite âme pour fournir ledit élément flexible.

8. Procédé selon la revendication 7, comprenant en outre l'étape de polymériser autour dudit premier composite un matériau polymérisable, capable de former ladite partie haptique, pour former une couche externe autour dudit premier composite.

9. Procédé selon la revendication 7, comprenant en outre le positionnement dudit premier composite dans un moule, la mise en place dudit matériau polymérisable dans ledit moule autour dudit composite, et la polymérisation dudit matériau polymérisable.

10. Procédé selon la revendication 7, dans lequel ladite surface est hydrolysée par réaction avec un acide fort ou une base forte.

11. Procédé selon la revendication 6, dans lequel ledit matériau à réfraction élevée est choisi dans le groupe constitué du polyméthacrylate de méthyle et des copolymères de monomères à indice de réfraction élevé choisis dans le groupe constitué de l'acrylate de 2-phénoxyéthyle, du méthacrylate de 2-phénoxyéthyle, de l'acrylate de benzyle, du méthacrylate de benzyle, de l'acrylate de 2-phényléthyle, du méthacrylate de 2-phényléthyle, du N-vinylcarbazole, de l'acrylate de 3-phénylpropyle, et du méthacrylate de 3-phénylpropyle.

12. Procédé selon la revendication 2, dans lequel ledit élément flexible est choisi dans le groupe constitué de l'homopolymère de polyacrylate d'éthyle, du poly (acrylate d'hydroxyéthyle) hydraté, des polymères et des copolymères d'acrylate de n-propyle, d'acrylate de n-butyle, d'acrylate de n-hexyle, de N-vinylpyrrolidone hydraté, de méthacrylate de 3-hydroxypropyle hydraté, d'acide méthacrylique hydraté, d'acide acrylique hydraté, de poly(diméthyl-diphényl-siloxane) faiblement réticulé et de poly(diméthyl-siloxane) faiblement réticulé.

13. Procédé selon la revendication 1, dans lequel ladite partie haptique est choisi dans le groupe constitué du polyméthacrylate de méthyle, des copolymères réticulés comprenant des monomères acrylates/méthacrylates ayant une Tg d'au moins 40 °C et des copolymères de diméthyl-diphénylpolysiloxane réticulé.

14. Procédé selon la revendication 1, dans lequel ledit élément flexible est plus flexible que ladite partie optique et ladite partie haptique.

15. Procédé selon la revendication 1, dans lequel ladite partie optique est du polyméthacrylate de méthyle.

16. Procédé selon la revendication 1, dans lequel ledit élément flexible est choisi dans le groupe constitué du polyacrylate d'éthyle et du polyacrylate d'hydroxyéthyle hydraté.

17. Procédé selon la revendication 1, dans lequel ladite partie haptique est du polyméthacrylate de méthyle.

18. Procédé selon la revendication 5, comprenant en outre la fourniture dudit second composite sous la forme d'une tige, ledit matériau pouvant être utilisé comme ladite partie haptique constituant une couche externe autour dudit premier composite.

19. Procédé selon la revendication 18, comprenant en outre la coupe d'un disque à partir de ladite tige et le retrait d'une partie de ladite couche externe de ladite tige pour former au moins une partie haptique.
